# EUROPÄISCHE PATENTSCHRIFT

(11) **EP 1 831 608 B1**
(45) Veröffentlichungstag und Bekanntmachung des Hinweises auf die Patenterteilung: **14.12.2011**
(21) Anmeldenummer: 05821554.2
(22) Anmeldetag: 22.12.2005
(51) Int. Cl.: F24C 7/08

(54) **GARGERÄT MIT MINDESTENS EINEM GASSENSORARRAY, PROBENAHMESYSTEM FÜR SOLCH EIN GERÄT, VERFAHREN ZUM GAREN MIT SOLCH EINEM GARGERÄT UND VERFAHREN ZUM REINIGEN SOLCH EINES GARGERÄTS**
COOKING APPLIANCE COMPRISING AT LEAST ONE GAS SENSOR ARRAY, SAMPLING SYSTEM FOR SUCH A COOKING APPLIANCE, METHOD FOR COOKING USING SAID COOKING APPLIANCE AND METHOD FOR CLEANING SAID COOKING APPLIANCE
APPAREIL DE CUISSON COMPORTANT AU MOINS UN RESEAU DE CAPTEURS DE GAZ, SYSTEME D'ECHANTILLONNAGE POUR UN TEL APPAREIL DE CUISSON, PROCEDE DE CUISSON METTANT EN OEUVRE UN TEL APPAREIL DE CUISSON ET PROCEDE DE NETTOYAGE D'UN TEL APPAREIL DE CUISSON

(30) Priorität: 27.12.2004 DE 102004062737
(43) Veröffentlichungstag der Anmeldung: 12.09.2007
(73) Patentinhaber: Rational AG, 86899 Landsberg/Lech (DE)
(72) Erfinder: STERZEL, Roland, 60437 Frankfurt/Main (DE); GREINER, Michael, 86899 Landsberg/Lech (DE); JÜRGENS, Andrea, 85551 Kirchheim (DE); IMGRAM, Judith, 63456 Hanau (DE); KLASMEIER, Jürgen, 86899 Landsberg/Lech (DE); LAUTERBACH, Katrin, 86899 Landsberg/Lech (DE); HILDENBRAND, Kathrin, 91616 Wachsenberg (DE); MAAS, Bruno, 86916 Kaufering (DE); SCHULLER, Erwin, 82515 Wolfratshausen (DE)
(74) Vertreter: Weber-Bruls, Dorothée
(86) Internationale Anmeldenummer: PCT/DE2005/002311
(87) Internationale Veröffentlichungsnummer: WO 2006/069563

(56) Entgegenhaltungen:
- DE-C1- 10 114 080
- US-A- 5 386 099
- PATENT ABSTRACTS OF JAPAN Bd. 015, Nr. 113 (M-1094), 18. März 1991 (1991-03-18) & JP 03 005622 A (OMRON TATEISI ELECTRON CO), 11. Januar 1991 (1991-01-11)
- PATENT ABSTRACTS OF JAPAN Bd. 016, Nr. 145 (M-1233), 10. April 1992 (1992-04-10) & JP 04 003812 A (MITSUBISHI ELECTRIC HOME APPLIANCE CO LTD; others: 01), 8. Januar 1992 (1992-01-08)
- PATENT ABSTRACTS OF JAPAN Bd. 017, Nr. 237 (M-1408), 13. Mai 1993 (1993-05-13) & JP 04 363516 A (SHARP CORP), 16. Dezember 1992 (1992-12-16)

## Beschreibung

Die vorliegende Erfindung betrifft ein Probennahmesystem für ein Gargerät nach dem Oberbegriff von Anspruch 1 und ein Gargerät mit solch einem Probennahmesystem. Ferner betrifft die Erfindung ein Verfahren zum Garen mit dem Erfindungsgemäßen Gargerät.

Den Garverlauf von Gargütern exakt verfolgen zu können, um zum Beispiel den gewünschten Garendzustand bestimmen und das Gargut rechtzeitig aus dem Gargerät entnehmen zu können, ist insbesondere für Großküchen sowie im Kantinenbetrieb von großer Bedeutung. Denn werden die gewünschten Garendzustände nicht realisiert, weist das Gargut Häufig geschmackliche Defizite, zum Beispiel einen zu starken Bräunungsgrad, auf und ist im Extremfall komplett zu verwerfen. Bei großen Gargutchargen, wie sie in Großküchen üblich sind, geht hiermit kein geringer wirtschafticher Schaden einher. Häufige Ursache ist, dass sich Garverläufe nicht vollständig standardisiern lassen, was wiederum zurückgeführt werden kann auf die uneinheitliche Größe von Gargütern, unterschiedliche Ausgangsgarzustände und die selten völlig übereinstimmende Gesamtmenge an zu garenden Gargütern.

Um dennoch unabhängig von der Art, Größe und Anzahl der eingesetzten Gargüter zu reproduzierbaren Garergebnissen zu gelangen, greift man vermehrt auf sogenannte Garprozeßfühler, zum Beispiel in Form von Kerntemperaturfühlern, zurück. Solche Garprozeßfühler finden sich zum Beispiel in der DE 202 04 393 U 1, DE 299 23 215 U 1 oder der DE 199 45 021 beschrieben. Mit Hilfe dieser Kerntemperaturfühler kann unter Verwendung vorgegebener Leitwerte festgestellt werden, wann ein Gargut während eines Garprozesses in seinem Kern die gewünschte vorgegebene Soll-Gartemperatur erreicht hat. Hierfür ist zumeist erforderlich, dass der Kerntemperaturfiihler in der Weise mechanisch in das Gargut eingesteckt wird, dass er tatsächlich bis in das Zentrum desselben gelangt. Selbstverständlich wird bei dieser Art der Messung das Gargut durch den Einstechvorgang partiell zerstört. Häufig ist auch nach Beendung des Garvorgangs auf der Oberfläche des Garguts die Einstichstelle noch zu erkennen. Da der Kerntemperaturfühler sich nicht selten während des gesamten Garvorgangs im Garinnenraum befindet, kommt es zuweilen aufgrund von Unachtsamkeit zu Verletzungen des Bedienpersonals. Auch gelingt während des Garverlaufs ein Umstecken des Garprozessfiihlers nicht immer optimal, so dass beispielsweise der Garprozeßfühler versetzt zum eigentlichen Zentrum des Garguts eingesteckt wird. Ferner kann es vorkommen, dass sich der Kemtemperaturfühler aufgrund des geringen Querschnitts des Garguts überhaupt nicht in demselben plazieren läßt. Auch ist die Kerntemperatur nicht notwendigerweise repräsentativ für den Garzustand, d.h. eine Korrelation zwischen der Kerntemperatur und dem Garzustand ist nur bei genauer Kenntnis des Garguts möglich.

Gegenwärtig wird ebenfalls versucht, den Garzustand von Lebensmitteln mit Hilfe von Gassensoren zu ermitteln. Allerdings gehen diese Bemühungen derzeit nicht über das Projektstadium hinaus. Zum Beispiel soll in dem vom Bundesministerium für Bildung und Forschung der Bundesrepublik Deutschland geförderten Projekt "Sensorsystem zur Kontrolle von Brat-, Back- und Röstvorgängen mittels Primäraromastandards" ermittelt werden, ob sich mit Hilfe geeigneter Gassensoren der Garendzeitpunkt beim Garen von Lebensmitteln anhand des Geruchs des fertigen Lebensmittels feststellen läßt. Im Rahmen des genannten Projekts wurde des weiteren der Prozeß der Kaffeeröstung sowie die Warenkontrolle von Lebensmitteln mit Hilfe von Gassensoren untersucht. (s.a. www.mst-innovationen.de, Infobörse, Mikrosystemtechnik, 43-2003, L. Heinert, N. Telde, "Einsatz von Halbleitergassensoren zur Erkennung von Röst-, Brat- und Backvorgängen in der Lebensmittelindustrie (SPAN)"). Diese Dokument offenbart die Merkmale des Oberbegriffs des Anspruchs 1.

Bei der vorangehend genannten Methode nutzt man aus, dass Lebensmittel bei Erwärmung zwar zahlreiche flüchtige Substanzen freisetzen, jedoch regelmäßig nur wenige davon zu derem charakteristischen Geruch beitragen. Beispielsweise setzt sich der Geruch von Butter aus insgesamt 230 flüchtigen Substanzen zusammen, von denen allerdings nur insgesamt 19 als geruchsaktiv zu bezeichnen sind (L.M. Nijssen et al., Volatile Compound in Food, 7. ed., TNO Nutrition and Food Research Institute, Zeist, Niederlande). Derartige geruchsaktive Substanzen lassen sich mit Hilfe von Metalloxid-Gassensoren, zum Beispiel auf der Basis von Zinndioxid oder Zinkoxid, detektieren. (s.a. T. Hofmann et al., "High resolution gas chromatography/selective odorant measurement by multisensor array (HRGC/SOMSA): a useful approach to standardise multisensor arrays for use in the detection of key food odorants", Sensors and Actuators B 41 (1997), Seiten 8 bis 87). Das vorangehend beschriebene Verfahren basiert auf der Verwendung sogenannter Leitstrukturen. Die für den Geruch verantwortlichen, charakteristischen Komponenten bzw. Leitstrukturen sind hierbei von den zum Einsatz kommenden Gassensoren zu erkennen, um aus den detektierten Signalen die gewünschten Aussagen ableiten zu können. Dieses erfordert zumindest anfänglich den parallelen Einsatz eines HR-Gaschromatographen. Sobald das mittels Gaschromatographie ermittelte Leitstruktursignal dem korrespondierenden Signal des Gassensors zuordbar ist, kann auf den Einsatz des Gaschromatographen verzichtet werden.

Wie von Lemme in Elektronik/17 2002, Seiten 42 bis 48, beschrieben wird, entfällt bei Verwendung eines Gassensorarrays mit der Bezeichnung KAMINA des Forschungszentrums Karlsruhe die vorherige Feststellung von Leitkomponenten. Vielmehr werden einfach nur die mit diesem Gassensor detektierten Muster gegeneinander abgeglichen. Angebracht über einer Bratpfanne, die mit Steaks gefüllt ist, sollen sich mit dem genannten KAMINA-Sensorarray nach einer anfänglichen Lernphase die verschiedenen Bratzustände von "raw" über "medium" und "well done" bis "überbraten" ermitteln lassen. Die Beheizung der Pfanne soll sich mit Hilfe des genannten Sensorarrays automatisch genau im gewünschten Moment abschalten. Hinweise zur weitergehenden Eignung des beschriebenen Sensorarrays für die Bestimmung von Garzuständen finden sich in der genannten Literaturstelle ebensowenig beschrieben wie in der dem genannten Gassensor zugrunde liegenden deutschen Patentschrift DE 44 23 289 C1. Es ist bereits fraglich, ob sich die Einsatzmöglichkeiten dieses Sensortyps auf völlig anders geartete Situationen erweitern lassen. Beispielsweise ist die Garungsatmosphäre im Innenraum von insbesondere industriellen Gargeräten nicht mit der über einer offenen Bratpfanne anzutreffenden Atmosphäre vergleichbar. Dieses trifft um so mehr auf sogenannte Heißluftgargeräte zu, in denen regelmäßig ein schnell rotierendes Lüfterrad mit zum Einsatz kommt. Auch ist die Luftfeuchtigkeit in solchen Geräten regelmäßig sehr hoch und befindet sich nicht selten im Sättigungsbereich. Bislang hat sich der Garvorgang in derartigen Heißluftdampfgeräten einigermaßen zufriedenstellend nur mit Hilfe von Kemtemperaturfühlern kontrollieren lassen. Demgemäß werden die in der US 6,784,404 und der US 2004/0144768 A1 bei Gargeräten zum Einsatz kommenden Gassensoren ausschließlich zur Bestimmung des Kohlenmonoxidgehaltes mit dem Ziel eingesetzt, über die Bestimmung des Verschmutzungsgrades des Garraums die Dauer des Reinigungszyklus eines selbstreinigenden Gargeräts festlegen zu können.

Es wäre daher wünschenswert, insbesondere auch aufgrund der bei Verwendung von Kerntemperaturenfühlern beobachteten Unzulänglichkeiten, auf ein Mittel zur Verfolgung des Garverlaufs zurückgreifen zu können, das reproduzierbar zuverlässige Ergebnisse liefert, ohne das Gargut zum Beispiel hinsichtlich Größe und Ausgangsgarzustand vorab spezifizieren zu müssen.

Aus der US 4,378,691 ist beispielsweise ein multifunktionaler Sensor bekannt, der ein einzelnes Sensierelement umfaßt, das durch ein Heizelement beheizt wird. Dieses Sensierelement kann dazu verwendet werden, in Abhängigkeit von der Feuchtigkeit in einem Garraum ein Gargerät zu steuern.

In der DE 103 07 247 A1 wird eine Dunstabzugshaube eines Elektroherds mit einem Abluftrohr offenbart, die eine Vielzahl von Sensoren aufweist, die zum Auswerten von gasförmigen Medien oder Stoffen in gasförmigen Medien ausgebildet sind. Anhand der über die Sensoren erfaßten sowie ausgewerteten Daten soll beispielsweise ein Gebläse in dem Abluftrohr des Elektroherds geregelt werden können. Gleichfalls kann gemäß der DE 103 07 247 A1 unter Zugrundelegung der erfaßten Meßdaten die Leistung des Elektroherds geregelt werden.

Die DE 10114 080 C1 offenbart ein Verfahren zum Bestimmen eine Garparameters aus einer organischen, flüssigen Substanz, die während eines Garprozesses von einem Gargut abgegeben wird, indem zumindest ein Sensor bereitgestellt wird, der eine spezifische Eigenschaft besagter Substanz, qualitativ und/oder quantitativ, erfasst, um auf der Grundlage besagten Garparameters eine Steuerung und/oder Regelung eines Garprozesses und/oder Reinigungsprozesses durchführen zu können.

Die US 6,170,318 B1 offenbart ein gattungsgemäßes Gargerät sowie ein gattungsgemäßes Probennahmesystem unter Einsatz eines Gassensorarrays mit einer Vielzahl von Gassensoren, das nach einer Lernphase verschiedene Stoffe detektieren können soll. Beispielsweise kann ein solches Gassensorarray in einem Mikrowellengerät oder an einem Bratgerät verwendet werden, um aufgrund der gemessenen Daten das entsprechende Gargerät zu steuern bzw. festzustellen, ob ein Gargut noch haltbar ist.

Die Aufgabe der vorliegenden Erfindung ist es daher, das gattungsgemäße Probennahmesystem derart weiterzuentwickein, dax die Nachteile des Stands der Technik überwunden werden. Insbesondere soll eine berührungslose Kontrolle eine Garzustands unabhängig von äußeren Störeinflüssen vor allem auch von größeren Gargutchargen im Innenraum eines Gargeräts erlaubt und eine größere Prozeßsicherheit gewährleistet werden.

Diese Aufgabe wird durch die Merkmale von Anspruch 1 gelöst.

Vorteilhafte Ausführungsformen des ertindungsgemäßen Probennahmesystems sind in den Ansprüchen 2 bis 8 beschrieben.

Ferner betrifft die vorliegende Erfindung ein Gargerät mit einem erfindungsgemäßen Probennahmesystem nach Anspruch 9. Bevorzugte erfinduagsgemäße Gargeräte sind in den Ansprüchen 10 bis 14 beschrieben.

Schließlich werden erfindungsgemäß noch ein Verfahren zum Garen von Gargut mit einem erfindungsgemäßen Gargerät mit den Ansprüchen 15 bis 22 beschrieben.

Grundsätzlich kommen sämtliche Gargeräte als Ausgangspunkt für die erfindungsgemäßen Gargeräte in Betracht, die aber einen Garinnenraum und einen Installationsraum verfügen. Besonders hervorzuheben sind solche Gargeräte, die darüber hinaus mit mindestens einem Belüftungssystem ausgestattet sind, d.h. sogenannte Heißluftdämpfer. Geeignete Heißlufdämpfer, die als Ausgangsbasis für die erfindungsgemäßen Gargeräte dienen, finden sich z.B. in der DE 196 515 14 A1 beschrieben. Die in konventionellen Heißluftdämpfern eingesetzten Lüfterräder erzeugen bei hohen Drehzahlen im Garinnenraum teilweise sehr hohe Luftgeschwindigkeiten, wodurch zuweilen auch Fett- und andere Flüssigkeitstropfen in dem Garinnenraum verwirbelt werden.

Selbstverständlich können eine Speicher- und Auswerteeinheit sowie ein Steuersystem eines Gargeräts gemäß der Erfindung in einem einzigen Prozessor vorliegen. Über das Steuersystem lassen sich z.B. die Garraumheizung, die Drehzahl eines Lüfterrads, der Dampfgenerator, die Belüftung, z.B. mit Frischluft, die Beschwadungsdüse und/oder die Reinigungsdüse ansteuern.

Ein erfindungsgemäßes Probenahmesystem, umfassend mindestens ein Gassensorarray, erlaubt die Überwachung der Gasatmosphäre im und am Gargerät und kann sich hierbei sowohl an Leitstrukturen, deren Signal zuvor ermittelt und in die Speichereinheit des Gargeräts eingegeben worden ist, als auch vorzugsweise an der Gesamtheit der detektierten Signal orientieren. In letzterem Fall werden die gewünschten Informationen ohne Verwendung einer Leitstruktur über die zeitliche Veränderung des komplexen Signalmusters während des Garverlaufs ermittelt, um z.B. Aussagen über den Garzustand machen zu können. Das heißt, es wird regelmäßig eine Vielzahl an von flüchtigen, insbesondere oxidierbaren und/oder reduzierbaren, Substanzen stammenden Signalen im Sinne eines Gesamtspektrums über die Gassensorarrays aufgenommen. Aus diesen Gesamtspektren sind Signale von bestimmten Einzelverbindungen in der Regel nicht zu extrahieren. Dieses ist aufgrund der Detektierung des Gesamtmusters über das Sensorarray im allgemeinen auch nicht erforderlich. Mindestens zwei, insbesondere eine Vielzahl an Einzelsensoren oder Sensorsegmenten eines Gassensorarrays liefern unter im wesentlichen identischen Bedingungen und bei im wesentlichen identischer zu messender Atmosphäre ein unterschiedliches Meßsignal. Auf diese Weise ergibt sich für jede spezifische Meßsituation bzw. Atmosphäre ein charakteristisches Gesamtmeßergebnis. Häufig reichen bereits 5 bis 100, beispielsweise auch 10 bis 50 Einzelsensoren oder Sensorsegmente aus für die Aufnahme von Signalmustern mit hinreichender Aussagekraft über einen Zeitabschnitt.

Um eine Aussage über einen Garzustand machen zu können, bedarf es im allgemeinen zunächst einer sogenannten Anlernphase. Die zu bestimmenden Zustände im Gargerät, insbesondere Garzustände, werden dabei zunächst experimentell abgefahren und die zeitliche Entwicklung der detektierten Signalmuster wird für einen optimalen Garverlauf als Normalzustand ermittelt und abgespeichert. Sodann werden zeitliche Verläufe von Signalmustern für von diesem optimalen Zustand abweichende Garverläufe ermittelt und abgespeichert. Bewegt sich demgemäß bei einem Garprozeß die zeitliche Veränderung der detektierten Signale innerhalb der gewünschten Grenzen bzw. Toleranzen, wird die vorgesehene, gewünschte Garprozeßsteuerung beibehalten. Andernfalls kommen Alternativlösungen zum Einsatz. Von Bedeutung ist somit insbesondere die Verfolgung der zeitlichen Entwicklung bzw. Veränderung der Gesamtheit der detektierten Signale. Im allgemeinen reicht es aus, obige Anlernphase für einen bestimmten Gargerätetyp einmal durchzuführen. Die erhaltenen Signalmuster können dann ohne weiteres für alle weiteren Gargeräte verwendet und z.B. in deren Speichereinheit hinterlegt werden.

Als besonders vorteilhaft hat sich erwiesen, dass mindestens ein Gassensorarray im Garinnenraum, im Installationsraum, im Belüftungssystem und/oder außerhalb des Gargeräts angebracht ist.

Ebenfalls bevorzugt umfaßt das Gassensorarray mehrere Felder aus einem halbleitenden Metalloxid-Film, welche jeweils mit zwei Elektroden verbunden sind, wobei die Felder eine im wesentlichen durchgehende Fläche bilden, die Elektroden eine bandförmige Gestalt aufweisen und die durchgehende Fläche in der Weise in die Felder aufteilen, dass jedes Feld in den durchgehenden Flächen jeweils durch die zwei Elektroden abgegrenzt ist.

Dementsprechend können geeignete Gassensorarrays eine Anordnung von mehreren, beispielsweise acht, Einzelsensoren umfassen, die paarweise auf einem Siliziumchip angeordnet sind. Jeder dieser Einzelsensoren besteht aus einem oder mehreren der halbleitenden Oxide SnO₂, ZnO, TiO₂ und WO₃ und ist als Dünnfilm auf dem Chip aufgetragen, welcher zumindest teilweise mit Palladium oder Platin als Katalysator überdeckt ist. Sämtliche eingesetzten Einzelsensoren unterscheiden sich in ihrer Zusammensetzung oder ihrem Aufbau. Bei Kontakt mit Meßgasen verändert sich die Leitfähigkeit der halbleitenden Oxide in Abhängigkeit von ihrer Zusammensetzung und der gegebenenfalls darauf angebrachten katalytisch wirkenden Palladiumbeschichtung, weshalb jeder Einzelsensor in Kontakt mit einem Meßgas ein unterschiedliches Signal liefert, das der Änderung der Leitfähigkeit proportional ist. Ein derartiges Gassensorarray findet sich z.B. bei X. Wang et al., Sensors and Actuators B 13 - 14 (1993) 458 - 461, beschrieben.

Besonders bevorzugt wird auf Gassensorarrays zurückgegriffen, bei denen unterschiedliche Felder, umfassend einen halbleitenden Metalloxid-Dünnfilm, die jeweils mit zwei Elektroden verbunden sind, in Abhängigkeit von Temperatur, Zusammensetzung, Dotierung und/oder Beschichtung bei Kontakt mit reduzierenden oder oxidierenden Gasen unterschiedliche Leitfähigkeitsänderungen aufweisen.

Die sensitive Schicht eines bevorzugten Gassensorarrays setzt sich im wesentlichen aus einer einzigen, durchgehenden Schicht aus einem oder mehreren halbleitenden Oxiden, beilspielsweise Zinndioxid, zusammen, wobei diese durchgehende Schicht durch bandförmige Elektroden in einzelne Felder unterteilt wird. Die Elektroden können direkt auf oder unter der Oberfläche der durchgehenden Schicht aufgetragen werden. Die durchgehende Fläche wird insbesondere in der Weise in die genannten Felder aufteilt, dass jedes Feld in der durchgehenden Fläche vorzugsweise jeweils durch die zwei Elektroden abgegrenzt ist. Gemäß einer vorteilhaften Ausführungsform ist der Gassensorarray mit einer Beschichtung versehen, deren Durchlässigkeit für reduzierende oder oxidierende Gase sich zwischen den beiden äußeren Elektroden stetig ändert.

Insbesondere bei der Analyse komplexer Gassysteme ist es von Vorteil, wenn sich die einzelnen Felder des Sensorarrays in ihrem Aufbau oder in ihrer Zusammensetzung voneinander unterscheiden. Jedes Feld wird folglich eine gegenüber den anderen Feldern unterschiedliche Leitfähigkeitsänderung hervorbringen, wenn der Sensor mit einem einzigen Gas in Kontakt gebracht wird. Eine unterschiedliche Zusammensetzung der Felder läßt sich z.B. durch Aufdampfen von Edelmetallen, d.h. eine Dotierung des Metalloxidfilms, über unterschiedlich lange Zeiträume erreichen. Eine kontinuierliche Veränderung der Zusammensetzung über die durchgehende Fläche des Gassensorarrays hinweg kann mit Hilfe der Gasphasenabscheidung (chemical vapor deposition) erreicht werden.

Auch kann es von Vorteil sein, die Empfindlichkeit eines Gassensorarrays für bestimmte Gase durch Anlegen bestimmter Temperaturen einzustellen, insbesondere durch Anlegen unterschiedlicher Temperaturen an die unterschiedlichen Felder. Hierzu ist vorab anzumerken, dass die Empfindlichkeit eines Gassensorarrays grundsätzlich dann hoch ist, wenn:
- eine starke Änderung in einem Signal des Gassensorarrays, zum Beispiel im Widerstand eines Einzelsensors, in Abhängigkeit von der Zeit einer ablaufenden chemischen Reaktion erkennbar ist;
- ein Signal des Gassensorarrays an sich stark ist, dass heißt eine definierte Konzentration ein möglich starkes Signal erzeugt;
- verschiedene, gleichzeitig entstehende Gase möglichst unterschiedlicher Signalmuster im Gassensorarray erzeugen, oder
- die Nachweisgrenze eines Gases so niedrig ist, dass die Identifikation des Gases bereits bei geringen Konzentrationen erfolgen kann.

Es hat sich gezeigt, dass ein und derselbe Sensor eines Gassensorarrays je nach Temperatur gegensätzliche Empfindlichkeiten für verschiedene Gase zeigt, weshalb die Temperatur eines jeden Sensors vorteilhafterweise einstellbar sein sollte. Zu diesem Zwecke sollte die Temperatur jedes Feld des Gassensorarrays, beispielsweise über ein Thermoelement, erfaßt, und jedes Feld gezielt, beispielsweise über einen Heizdraht, aufgeheizt werden können.

Besonders geeignete Gassensorarrays finden sich z.B. in der DE 44 23 289 C1 beschrieben und sind auch als sogenannte Kamina-Sensoren des Forschungszentrums Karlsruhe bekannt.

Gemäß eine weiteren Aspek der vorliegenden Erfindung zeichnen sich die erfindungsgemäßen Probenahmesystem auch aus durch mindestens eine zweite Zuleitung für die Atmosphäre aus dem Installationsraum zu mindestens einem ersten, zweiten, dritten und/oder vierten Gassensorarray, mindestens eine dritte Zuleitung für die Atmosphäre aus dem Belüftungssystem zu mindestens einem ersten, zweiten, dritten und/oder vierten Gassensorarray und/oder mindestens eine vierte Zuleitung für die das Gargerät umgebende Atmosphäre zu mindestens einem ersten, zweiten, dritten und/oder vierten Gassensorarray.

Ferner sind geeignete Probenahmesyteme ausgestattet mit mindestens einer ersten Ableitung von dem ersten, zweiten, dritten und/oder vierten Gassensorarray.

Um die Meßoberfläche des Gassensorarrays vor dauerhafter Verschmutzung zu schützen, hat es sich als vorteilhaft erwiesen, mindestens einen Filter vor mindestens einem Gassensorarray, insbesondere vor dessen Meßoberfläche, und/oder in oder am Eingang der ersten, zweiten, dritten und/oder vierten Zuleitung anzubringen. Geeignete Filter stellen z.B. Kunststoffmembrane, beispielsweise aus Teflon, Keramikfilter, beispielsweise eine poröse Aluminiumoxidkeramik, oder metallische Filter, beispielsweise ein poröser Metallschaum, dar. Sintermetallfilter sind besonders geeignet.

Ferner zeichnen sich erfindungsgemäße Probenahmesysteme aus durch mindestens ein, insbesondere über die Steuereinheit ansteuerbares, Ventil am Eingang und/oder im Bereich der zweiten, dritten und/oder vierten Zuleitung und der Ableitung.

Mit Hilfe der genannten Ventile insbesondere im Verlauf der Ableitung kann z.B. der Eintrag von Abluft in das Gassensorarray verhindert werden. Die Zuleitungen zu den Gassensorarrays sind vorzugsweise sehr kurz ausgeführt, um das detektierte Signal nicht unnötig zu verzögern oder zu verschmieren.

Geeignete Probenahmesysteme umfassen in einer weiteren Ausführungsform zudem mindestens eine Pumpeneinheit in Wirkverbindung mit der ersten, zweiten, dritten und/oder vierten Zuleitung für den Transport von zu analysierender Atmosphäre zu dem/den Gassensorarray(s). Beispielsweise kann mit Hilfe einer Pumpe Atmosphäre aus dem Garraum oder dem Installationsraum oder gar Außenluft durch ein Filter, das die charakteristische Zusammensetzung des Probenvolumens nicht verändert, dem Gassensorarray zugeführt werden. Das Filter kann in diesem Fall z.B. Feststoffpartikel sowie Fett- und Flüssigkeitstropfen abhalten.

Erfindungsgemäß ist ebenfalls vorgesehen, dass mindestens zwei Zuleitungen direkt oder indirekt mit einem Gassensorarray verbunden sind.

Beispielsweise ist mindestens ein Gassensorarray in die Innenwand des Garinnenraums oder des Installationsraums integriert. Selbstverständlich kann zur Ermittlung bzw. Analyse der Atmosphäre im Bereich außerhalb des Gargeräts das Gassensorarray auch auf der Außenwand des Gargeräts vorliegen bzw. in diese integriert sein. Ferner können gemäß einer weiteren Ausführungsform auch die in der Innen- oder Außenwand des Gargeräts eingelassenen Gassensorarrays Zuleitungen zwecks Heranführung von zu analysierender Atmosphäre aufweisen. Dieses ist insbesondere dann von Vorteil, wenn das Gassensorarray nicht auf der Wandfläche aufliegt, sondern in diese integriert ist. Diese Zuleitungen können auch genutzt werden, um geeignete Filter vor dem Gassensorarray anzubringen.

Vorzugsweise sind mindestens zwei Innenwände des Garinnenraums mit mindestens einem Gassensorarray ausgestattet. Auf diese Weise läßt sich der Garverlauf ortsabhängig detektieren.

Besonders vorteilhaft ist es also, wenn bei einem Anlernen unterschiedliche Temperaturprofile an ein Gassensorarray angelegt werden, die Signale des Gassensorarrays abgespeichert werden und jedem Signalmuster des Gassensorarrays, das einen Standard bildet, ein Garprogramm zugeordnet wird. Im Normalbetrieb des erfindungsgemäßen Gargerätes werden dann Informationen von einer Bedienoberfläche desselben und dem Gassensorarray dazu benutzt, chemische Vorgänge, die im Garraum ablaufen, durch Vergleich mit besagten Standards zu klassifizieren. Sobald eine Klassifizierung stattgefunden hat, kann auf ein geeignetes, experimentell bestimmtes Temperaturprofil zurückgegriffen werden, das die Empfindlichkeit des Gassensorarrays optimiert und die Auswahl eines optimalen Garprogramms ermöglicht. Mit Hilfe einer Vielzahl von Thermoelementen und Heizelementen läßt sich das Temperaturprofil des Gassensorarrays jederzeit einstellen, durchaus auch mehrfach während eines Garprozesses, nämlich wenn eine Klassifizierung anzupassen ist.

Soll beispielsweise gemäß einer Eingabe über die Bedienoberfläche eines Gargerätes bei einem zeitgesteuerten Garprogramm ausschließlich die Feuchte im Garraum geregelt werden, so wird erfindungsgemäß das Temperaturprofil aus einer Vielzahl abgespeicherter Temperaturprofile automatisch ausgewählt, mit dem sich möglichst genau der H₂O-Gehalt der Garatmosphäre bestimmen läßt, während größere Kohlenwasserstoffe, die durch die Garung des Gargutes selbst verursacht werden, möglichst wenig zum Signal beitragen sollen.

Wird andererseits über die Bedienoberfläche eingegeben, dass Fleisch gebraten werden soll, und läßt sich über das Profil bzw. Muster des Gassensorarrays erkennen, dass es sich mit großer Wahrscheinlichkeit bei dem Fleisch um Rindfleisch handelt, so wird von der Steuereinrichtigung des erfindungsgemäßen Gargeräts automatisch ein Temperaturprofil ausgewählt, das sich in seiner Empfindlichkeit für gebratenes Rindfleisch bewährt hat. Dabei werden dann größere Kohlenwasserstoffe, die bei einer Garung auftreten, besonders stark zur Bildung des Signalmusters beitragen.

Mit den vorangehend geschilderten Ausfiihrungsformen des erfindungsgemäßen Gargeräts, Probennahmesystems oder Verfahrens ist es möglich, Gerüche an verschiedenen Orten im bzw. am Gargerät zu detektieren. Dieses kann entweder mit Hilfe mehrerer Gassensoren, gegebenenfalls ausgestattet mit eigenen Zuleitungen zu Probennahme, geschehen, die an den Meßorten angebracht sind, oder mit Hilfe einer Vielzahl an Zuleitungen, die sämtlich ein zentrales Gassensorarray bedienen.

Erfindungsgemäß gelingt ein optimales Garergebnis auch dann, wenn die Garinnenraumatmosphäre während des Garvorgangs, z.B. durch häufiges Öffnen und Schließen der Garraumtür, gestört bzw. verändert wird. Bei Veränderungen der Garraumatmosphäre, die dem Gargerät nicht bekannt sind bzw. nicht in der Speichereinheit hinterlegt sind, kann für das Bedienpersonal eine Fehlermeldung provoziert werden. Von Vorteil ist weiterhin, dass sich über die detektierten Geruchsmuster bei anfänglichem Aufheizen des Garguts dessen Ausgangszustand, z.B. tiefgekühlt, mariniert, etc., bestimmen läßt. Wird anhand der zeitlichen Entwicklung des detektierten Gesamtmuster z.B. ein tiefgekühltes Gargut festgestellt, kann zunächst eine Auftau-oder Aufwärmphase angesteuert werden. Wird marinierte Ware detektiert, läßt sich sicherstellen, dass diese nicht überhitzt wird. Auch läßt sich in diesem frühen Stadium des Garverfahrens ebenfalls feststellen, ob das zu garende Lebensmittel möglicherweise bereits verdorben ist oder ob schlecht gereiftes Fleisch gegebenenfalls einer Haltephase auszusetzen ist, um dennoch zu dem gewünschten Garresultat zu gelangen.

Von Vorteil ist somit, dass der Ausgangszustand des Garguts, z.B. des Fleisches, nicht mehr vom Anwender visuell oder haptisch festgestellt werden muss, sondern mit Hilfe des erfindungsgemäßen Gargeräts ermittelbar ist.

Da die Geschwindigkeit, mit der sich bekannte bzw. abgespeicherte Signalverläufe ändern, auch von der Menge der in den Garinnenraum eingebrachten Gargüter abhängt, läßt sich bereits in der Anfangsphase des Garprozesses mit den erfindungsgemäßen Gargeräten eine sogenannte Lasterkennung vornehmen. An die festgestellte Last kann sodann das Garprogramm individuell angepaßt werden.

Die Ermittlung geruchsaktiver Verbindungen kann ebenfalls eingesetzt werden, um sowohl den Oberflächenzustand wie auch die Gare des jeweiligen Garguts festzustellen. Ist beispielsweise ein Gargut bereits hinreichend gebräunt, die Gare jedoch noch nicht eingetreten, muss die Garraumtemperatur entsprechend verringert werden, damit nicht eine zu starke Bräunung eintritt. Aus der detektierbaren Geschwindigkeit der Oberflächenreaktion und der Geschwindigkeit der Gare läßt sich ferner auch die Stückgröße des Garguts ermitteln. Von Vorteil ist ferner, dass Vorgänge wie das Würzen, das Befeuchten und das Übergießen der Gargüter nicht mehr rein zeitabhängig vonstatten zu gehen hat, sondern in Abhängigkeit von dem tatsächlichen Garzustand zum jeweils korrekten Zeitpunkt vorgenommen werden kann. Dieser Vorgang kann mit Hilfe der erfindungsgemäßen Gargeräte selbstverständlich auch automatisiert werden.

Indem die Probennahme an mehreren Orten im Garinnenraum gleichzeitig oder nahezu gleichzeitig vorgenommen werden kann, erschließt sich dem Bedienpersonal mit den erfindungsgemäßen Gargeräten zudem ohne weiteres, ob das Gargut gleichmäßig gegart vorliegt oder ob es Bereiche mit stärker gegartem und mit weniger stark gegartem Gargut gibt. Über lenk- bzw. einstellbare Lenkbleche oder Abweiser lassen sich dann z.B. in einem Heißluftdämpfer die weniger stark gegarten Gargüter gezielt mit Energie beaufschlagen.

Mit Hilfe der in dem erfindungsgemäßen Probenahmesystem zum Einsatz kommenden Gassensorarrays läßt sich nach beendetem Garvorgang ebenfalls der Verschmutzungsgrad des Garraums ermitteln. Beispielsweise ist ein einfacher Abgleich von gespeichertem Anfangs- und Endzustand hierfür heranziehbar. Anhand dieses Verschmutzungsgrades kann durch das Gargerät automatisch ein an diesen Verschmutzungsgrad angepaßtes Reinigungsprogramm vorgeschlagen bzw. durchgeführt werden. Wird beispielsweise ein hoher Fettanteil detektiert, kann automatisch eine entsprechend hohe Menge an Emulgatoren vorgeschlagen bzw. eingesetzt werden. In gleicher Weise kann, wird eine eiweißhaltige Verschmutzung detektiert, ein Reinigungsmittel, enthaltend Enzyme, vorgeschlagen werden.

Außerdem sind mit dem erfindungsgemäßen Probenahmesystem Fehlbedienungen auszuschließen sowie Störungen, beispielsweise Schwelbrandgerüche, Überhitzung oder Leckagen des Garsystems im Installations- sowie im Garraum unmittelbar feststellbar. Insbesondere hat sich als vorteilhaft erwiesen, dass sich der Ausgangsgarzustand und der Endgarzustand bestimmen und gegeneinander abgleichen lassen, was einen Einblick dahingehend liefert, ob alle gewünschten Hygienevoraussetzungen eingehalten worden sind.

Wird beispielsweise ein Dampfgenerator in einem Gargerät eingesetzt, läßt sich mit Hilfe der in dem erfindungsgemäßen Gargerät vorliegenden Gassensorarrays auch die Qualität des Wassers unmittelbar feststellen.

Eine zuverlässige Datenaufnahme wird insbesondere durch den kombinierten Einsatz von mindestens einer Pumpe, mindestens einem Filter und mindestens einem Ventil gewährleistet.

Weitere Merkmale und Vorteile der Erfindung ergeben sich aus der nachstehenden Beschreibung, in der Ausführungsbeispiele eines erfindungsgemäßen Probenahmesystems anhand von schematischen Zeichnungen im Einzelnen erläutert werden. Dabei zeigt:
- Figur 1: eine schematische Schnittdarstellung eines erfindungsgemäßen Probenahmesystems;
- Figur 2: eine schematische Schnittansicht einer alternativen Ausführungsform eines erfindungsgemäßen Probenahmesystems; und
- Figur 3: eine Darstellung eines Gassensorarrays in Verbindung mit wesentlichen Komponenten eines erfindungsgemäßen Probenahmesystems.

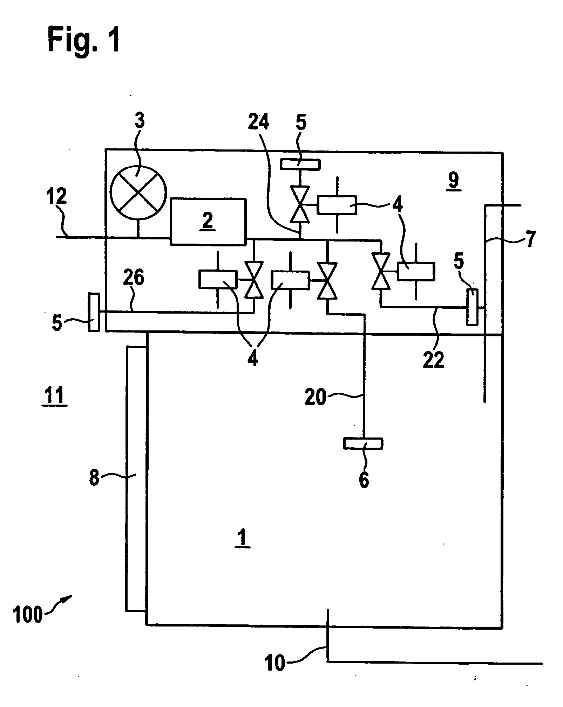

Figur 1 ist ein erfindungsgemäßes Gargerät in Form eines Heißluftdämpfers 100, umfassend einen Garraum 1 mit einer Garraumtür 8 und einem Abfluß 10, ein Belüftungssystem 7 sowie einen Installationsraum 9 zu entnehmen. In dieser Ausführungsform befindet sich ein Gassensorarray 2 im Installationsraum 9. Über Zuleitungen 20, 22, 24 und 26 kann dem Gassensorarray 2 eine zu analysierende Gasprobe aus dem Garraum 1, dem Belüftungssystem 7, dem Installationsraum 9 bzw. der Atmosphäre 11 außerhalb des Gargeräts 100 separat oder gleichzeitig zugeführt werden. Die Probennahme läßt sich mit Hilfe ansteuerbarer Ventile 4 sowie einer Pumpe 3, die dem Gassensorarray 2 nachgeschaltet ist, ohne weiteres bewerkstelligen. Es hat sich als vorteilhaft erwiesen, die Zuleitungen 20, 22, 24, 26 mit geeigneten Filtern 5 bzw. 6 auszustatten: Beispielsweise kann für die Messung der Atmosphäre im Garraum 1 vorgesehen sein, bis auf das Ventil 4 der Zuleitung 20 sämtliche weiteren Ventile 4 zu schließen, so dass nur die gewünschte Garraumatmosphäre dem Gassensorarray 2 zugeführt wird.

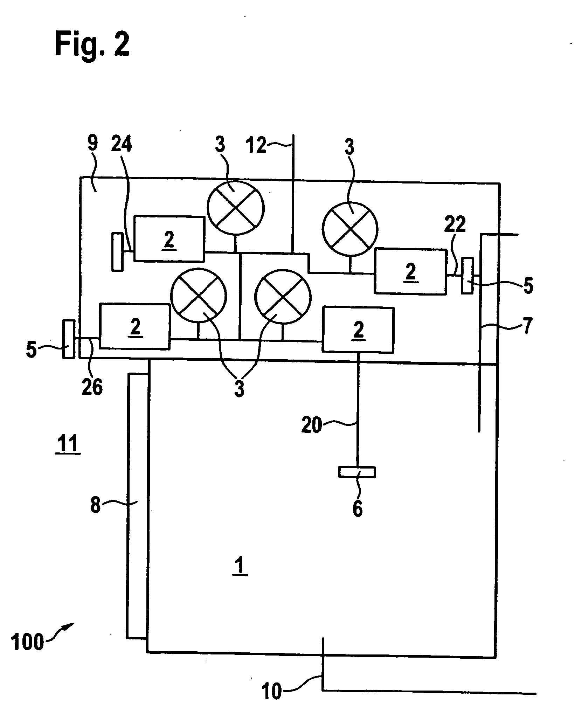

Alternativ kann, wie in Figur 2 gezeigt, jedes Probennahmesystem mit einem eigenen Gassensorarray 2 ausgestattet sein. Bei dieser Variante kann Atmosphäre aus dem Garraum 1 über die Zuleitung 20, Atmosphäre aus dem Belüftungssystem 7 über die Zuleitung 22, Atmosphäre aus dem Installationsraum 9 über die Zuleitung 24 und Atmosphäre 11 außerhalb des Gargeräts 100 über die Zuleitung 26 dem jeweiligen Gassensorarray 2 zugeführt werden. Wie bereits zu Figur 1 erläutert, wird auch bei der in Figur 2 dargestellten Variante mit Filtern 5 bzw. 6 im Bereich der Einlässe der Zuleitungen 20, 22, 24 und 26 gearbeitet. Mit Hilfe separat ansteuerbarer Pumpen 3 kann die Probenzuführung für jedes Gassensorarray 2 gesteuert werden.

Wie den Ausführungsformen der Figuren 1 und 2 zu entnehmen ist, können Gasproben ohne weiteres an verschiedenen Orten im oder am Gargerät 100 aufgenommen werden und entweder einem zentralen oder aber separat mehreren Gassensorarrays 2 zugeführt werden. Indem die Proben sowohl aus dem Innen- wie auch dem Außenraum des Gargeräts 100 dem bzw. den Gassensorarray(s) 2 zugeführt und vermessen werden können, lassen sich z.B. Störungen durch Umgebungslufteinflüsse bei der Bestimmung des optimalen Garverlaufs vermeiden.

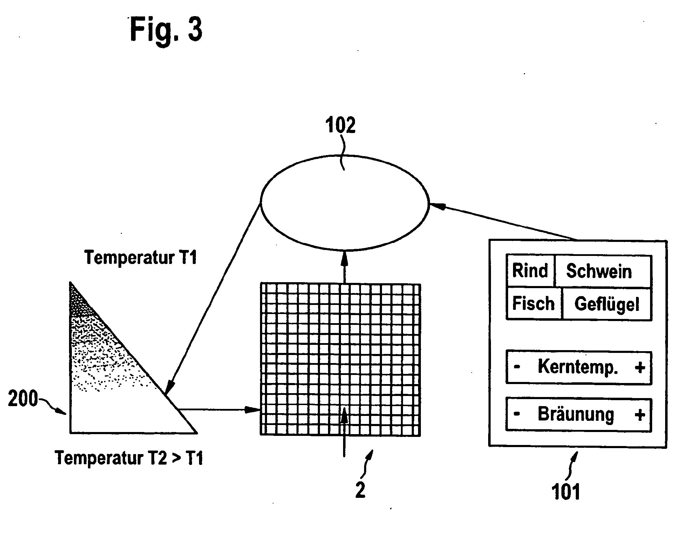

Wie Figur 3 zu entnehmen ist, kann ein Gassensorarray 2, wie es in einem Gargerät 100 gemäß Figur 1 oder 2 einsetzbar ist, eine Vielzahl von Feldern aufweisen, die jeweils dem Erfassen eines Gases dienen, wobei die Empfindlichkeit auf ein spezielles Gas über ein jeweils speziell aufgeprägtes Temperaturprofil einstellbar ist. Das Temperaturprofil ist dabei in Abhängigkeit von einer Gargutart, wie Rind, Schwein, Fisch oder Geflügel, einem Gargrad, bestimmt durch die Kerntemperatur und/oder die Bräunung, oder dergleichen, beispielsweise über eine Bedienoberfläche 101 des Gargeräts 100, auswählbar oder einstellbar, und zwar vorzugsweise unter Zwischenschaltung einer Steuer- oder Regeleinrichtung 102 des Gargeräts 100. Eine Nachregelung des Temperaturprofils 200 ist dann auch in Abhängigkeit von den Ausgabedaten des Gassensorarrays 2 selbst möglich. So kann es während eines Garverfahrens zu einer Optimierung der Empfindlichkeit und somit zu einer Optimierung der Zuordnung eines Garprozesses zu einem Standardgarprozeß und schließlich einem speziellen Garprogramm kommen, was schlußendlich das Erhalten reproduzierbar guter Garergebnisse sicherstellt.

### Bezugszeichenliste

- 1: Garraum
- 2: Gassensorarray
- 3: Pumpe
- 4: Ventil
- 5: Filter
- 6: Filter
- 7: Belüftungssystem
- 8: Garraumtür
- 9: Installationsraum
- 10: Abfluß
- 11: Atmosphäre
- 12: Ableitung
- 20: Zuleitung
- 22: Zuleitung
- 24: Zuleitung
- 26: Zuleitung
- 100: Gargerät, Heißluftdämpfer
- 101: Bedienoberfläche
- 102: Steuer- oder Regeleinrichtung
- 200: Temperaturprofil

## Patentansprüche

1. Probennahmesystem eines Gargeräts, umfassend mindestens ein Gassensorarray, dem über mindestens eine Zuleitung Atmosphäre zwecks Aufnahme von Signalmustern zuleitbar ist,
mindestens ein erstes Gassensorarray (2) zur Detektion der Atmosphäre aus einem Garraum (1) des Gargeräts (100),
ein zweites Gassensorarray (2) zur Detektion der Atmosphäre aus einem Installationsraum (9) des Gargeräts (100), ein drittes Gassensorarray (2) zur Detektion der Atmosphäre aus einem Belüftungssystem (7) des Gargeräts (100) und/oder ein viertes Gassensorarray (2) zur Detektion der das Gargerät (100) umgebenden Atmosphäre (11), **gekennzeichnet durch**
mindestens eine erste Zuleitung (20) für die Atmosphäre aus dem Garraum (1) zu dem ersten, zweiten, dritten und/oder vierten Gassensorarray (2), mindestens eine zweite Zuleitung (24) für die Atmosphäre aus dem Installationsraum (9) zu dem ersten, zweiten, dritten und/oder vierten Gassensorarray (2),
mindestens eine dritte Zuleitung (22) für die Atmosphäre aus dem Belüftungssystem (7) zu dem ersten, zweiten, dritten und/oder vierten Gassensorarray (2) und/oder mindestens eine vierte Zuleitung (26) für die das Gargerät (100) umgebende Atmosphäre (11) zu dem ersten, zweiten, dritten und/oder vierten Gassensorarray (2),
wobei
mindestens ein anstenerbaves Ventil (4) am Eingang der ersten, zweiten, dritten und/oder vierten Zuleitung (20, 22, 24, 26) angeordnet ist.

2. Probennahmesystem nach Anspruch 1, **gekennzeichnet durch** mindestens eine erste Ableitung (12) von dem ersten, zweiten, dritten und/oder vierten Gassensorarray (2), wobei vorzugsweise mindestens ein Ventil am Eingang der Ableitung angeordnet ist.

3. Probennahmesystem nach Anspruch 1 oder 2, **gekennzeichnet durch** mindestens einen Filter (5, 6) vor mindestens einem Gassensorarray (2), insbesondere vor dessen Meßoberfläche, und/oder in oder am Eingang der ersten, zweiten, dritten und/oder vierten Zuleitung (20, 22, 24, 26).

4. Probennahmesystem nach einem der Ansprüche 1 bis 3, **gekennzeichnet durch** mindestens eine Pumpeneinheit (3) in Verbindung mit der ersten, zweiten, dritten und/oder vierten Zuleitung (20, 22, 24, 26) für den Transport von zu analysierender Atmosphäre zu dem/den Gassensorarray(s) (2).

5. Probennahmesystem nach einem der Ansprüche 1 bis 4, **dadurch gekennzeichnet, dass**
das Gassensorarray (2) mehrere Felder aus einem halbleitenden Metalloxid-Film, welche jeweils mit zwei Elektroden verbunden sind, umfaßt, wobei die Felder eine im wesentlichen durchgehende Fläche bilden, die Elektroden eine bandförmige Gestalt aufweisen und die durchgehende Fläche in der Weise in die Felder aufteilen, dass jedes Feld in den durchgehenden Flächen jeweils durch die zwei Elektroden abgegrenzt ist.

6. Probennahmesystem nach einem der Ansprüche 1 bis 5, **dadurch gekennzeichnet, dass**
unterschiedliche Sensoren, Sensorsegmente und/oder Felder jedes Gassensorarrays (2) in Abhängigkeit von Temperatur, Zusammensetzung, Dotierung und/oder Beschichtung bei Kontakt mit reduzierenden oder oxidierenden Gasen unterschiedliche Leitfähigkeitsänderungen aufweisen.

7. Probennahmesystem nach Anspruch 6, **dadurch gekennzeichnet, dass** die Temperatur jedes Sensors, Sensorsegments und/oder Feldes des Gassensorarrays (2) einstellbar ist, vorzugsweise ein bestimmter Temperaturgradient oder ein bestimmtes Temperaturprofil auf das Gassensorarray (2) aufprägbar ist.

8. Probennahmesystem nach Anspruch 6 oder 7, **dadurch gekennzeichnet, dass** jeder Sensor, jedes Sensorsegment und/oder jedes Feld in Wirkverbindung mit einem, vorzugsweise steuerbaren, Thermoelement und/der Heizelement steht.

9. Gargerät (100), umfassend mindestens einen Garraum (1), mindestens einen Installationsraum (9), ein Belüftungssystem, ein Probennahmesystem nach einem der vorangehenden Ansprüche, mindestens eine Speichereinheit zur Speicherung der über das erste, zweite, dritte und/oder vierte Gassensorarray (2) detektierten Signale, mindestens eine Auswerteeinheit zur Verarbeitung der detektierten Signale, und mindestens eine Steuereinheit zur Steuerung von Garprozessen oder Reinigungsprozessen in Abhängigkeit von den ausgewerteten Signalen..

10. Gargerät nach Anspruch 9, **dadurch gekennzeichnet, dass** mindestens ein Gassensorarray (2) im Garraum (1), im Installationsraum (9), im Belüftungssystem (7) und/oder außerhalb des Gargeräts (100) angebracht ist, und/oder
mindestens ein Gassensorarray (2) in die Innenwand des Garraums (1), des Installationsraums (9) und/oder in die Außenwand des Gargeräts (100) integriert ist, wobei
vorzugsweise in mindestens zwei Innenwänden des Garraums (1) mindestens ein Gassensorarray (2) integriert ist.

11. Gargerät (100) nach Anspruch 9 oder 10, **dadurch gekennzeichnet, dass** die Speichereinheit, die Auswerteeinheit und/oder die Steuereinheit im Installationsraum (9), vorzugsweise in eine Steuer- oder Regeleinrichtung (102) integriert, angeordnet ist bzw. sind.

12. Gargerät (100) nach einem der Ansprüche 9 bis 11, **dadurch gekennzeichnet, dass**
die Temperatur, der Temperaturgradient oder das Temperaturprofil vor oder während eines Garprozesses oder Reinigungsprozesses variierbar ist.

13. Gargerät (100) nach einem der Ansprüche 9 bis 12, **dadurch gekennzeichnet, dass**
zumindest ein Thermoelement und/oder zumindest ein Heizelement einem Sensor, Sensorsegment und/oder Feld des Gassensorarrays (2) zugeordnet und/oder über die Steuereinheit ansteuerbar ist bzw. sind und/oder jedes Ventil (4) über die Steuereinheit ansteuerbar ist.

14. Gargerät (100) nach einem der Ansprüchen 9 bis 13, **dadurch gekennzeichnet, dass** dieses ein Heißluftdampfgargerät darstellt.

15. Verfahren zum Garen von Gargut mit einem Gargerät nach einem der Ansprüche 9 bis 14, **dadurch gekennzeichnet, dass**
zumindest Garraumatmophäre mindestens einem Sensor, Sensorsegment oder Feld zumindest eines Gassensorarrays zugeführt und intervallweise oder kontinuierlich während des Garens detektiert wird, das Analyseergebnis in der Auswerteeinheit mit einem in der Speichereinheit hinterlegten Standard abgeglichen wird, und der Garprozess in Abhängigkeit von dem Analyseergebnis geführt wird.

16. Verfahren nach Anspruch 15, **dadurch gekennzeichnet, dass** die Analyseergebnisse von einem ausgewählten Standard nicht oder nur innerhalb einer vorgegebenen Bandbreite abweichen.

17. Verfahren nach Anspruch 15 oder 16, **dadurch gekennzeichnet, dass** die Temperatur des Sensors, Sensorsegments oder Feldes und/oder der zum Vergleich herangezogene Standard variiert wird bzw. werden, insbesondere vor oder während des Garprozesses.

18. Verfahren nach einem der Ansprüche 15 bis 17, **dadurch gekennzeichnet, dass** Standards in einer Anlernphase in Form von Profilen oder Mustern der detektierten Signale jedes Gassensorarrays, insbesondere in Abhängigkeit von Gargutart, Gargutmenge, Gargutqualität und/oder gewünschtem Gargrad, vorzugsweise für unterschiedliche Temperaturen jedes Sensors, Sensorsegments und/oder Feldes, abgespeichert werden.

19. Verfahren nach einem der Ansprüche 15 bis 18, **dadurch gekennzeichnet, dass** nach Einbringen von Gargut in den Innenraum des Gargeräts, insbesondere während einer ersten Aufwärmphase, über das bzw. die Gassensorarray(s) die Art und/oder der Ausgangszustand des Garguts ermittelt wird bzw. werden.

20. Verfahren nach Anspruch 19, **dadurch gekennzeichnet, dass** die ermittelte Art und/oder der ermittelte Ausgangszustand bei der Steuerung des Garprozesses berücksichtigt wird bzw. werden.

21. Verfahren nach Anspruch 19 oder 20, **dadurch gekennzeichnet, dass**,
wenn der Ausgangszustand eines Garguts als verdorben qualifiziert wird, der Garprozeß gestoppt und/oder ein Warnsignal gegeben wird.

22. Verfahren nach einem der Ansprüche 15 bis 21, **dadurch gekennzeichnet, dass** jedem Standard in einer Anlernphase ein Garprogramm zugeordnet wird.

## Claims

1. A sampling system of a cooking device, comprising at least one gas sensor array that can be supplied with atmosphere through at least one supply pipe in order to sense signal patterns,
at least one first gas sensor array (2) to detect the atmosphere from a cooking chamber (1) of the cooking device (100),
a second gas sensor array (2) to detect the atmosphere from an installation compartment (9) of the cooking device (100), a third gas sensor array (2) to detect the atmosphere from a ventilation system (7) of the cooking device (100), and/or a fourth gas sensor array (2) to detect the atmosphere (11) surrounding the cooking device (100), **characterized by**
at least one first supply pipe (20) for the atmosphere from the cooking chamber (1) to the first, second, third and/or fourth gas sensor array (2), at least one second supply pipe (24) for the atmosphere from the installation compartment (9) to the first, second, third and/or fourth gas sensor array (2),
at least one third supply pipe (22) for the atmosphere from the ventilation system (7) to the first, second, third and/or fourth gas sensor array (2), and/or at least one fourth supply pipe (26) for the atmosphere (11) surrounding the cooking device (100) to the first, second, third and/or fourth gas sensor array (2), wherein
at least one controllable valve (4) is arranged at the inlet of the first, second, third and/or fourth supply pipe (20, 22, 24, 26).

2. A sampling system according to Claim 1, **characterized by**
at least one first discharge pipe (12) from the first, second, third and/or fourth gas sensor array (2), wherein preferably at least one valve is arranged at the inlet of the discharge pipe.

3. A sampling system according to Claim 1 or 2, **characterized by**
at least one filter (5, 6) in front of at least one gas sensor array (2), in particular in front of the measuring surface of said gas sensor array, and/or in or at the inlet of the first, second, third and/or fourth supply pipe (20, 22, 24, 26).

4. A sampling system according to any one of Claims 1 to 3, **characterized by**
at least one pumping unit (3) that is connected to the first, second, third and/or fourth supply pipe (20, 22, 24, 26) and serves to convey the atmosphere to be analyzed to the gas sensor array(s) (2).

5. A sampling system according to any one of Claims 1 to 4, **characterized in that**
the gas sensor array (2) comprises several sections that are made of a semiconducting metal oxide film and each of which is connected to two electrodes, wherein the sections form a substantially continuous surface, the electrodes are band-shaped and divide the continuous surface into the sections in such a manner that each section within the continuous surfaces is delimited by the two electrodes.

6. A sampling system according to any one of Claims 1 to 5, **characterized in that**
the conductivity of different sensors, sensor segments and/or sections of each gas sensor array (2) changes differently, in accordance with the temperature, composition, doping and/or coating, when said sensors, sensor segments and/or sections come in contact with reducing or oxidizing gases.

7. A sampling system according to Claim 6, **characterized in that**
the temperature of each sensor, sensor segment and/or section of the gas sensor array (2) can be adjusted, preferably a specific temperature gradient or a specific temperature profile can be permanently defined on the gas sensor array (2).

8. A sampling system according to Claim 6 or 7, **characterized in that**
each sensor, each sensor segment and/or each section is operatively connected to a, preferably controllable, thermocouple and/or heating element.

9. A cooking device (100) comprising at least one cooking chamber (1), at least one installation compartment (9), a ventilation system, a sampling system according to any one of the preceding claims, at least one memory unit to store the signals detected by means of the first, second, third and/or fourth gas sensor array (2), at least one evaluation unit to process the signals that have been detected, and at least one control unit to control cooking processes or cleaning processes in accordance with the signals that have been evaluated.

10. A cooking device according to Claim 9, **characterized in that**
at least one gas sensor array (2) is mounted in the cooking chamber (1), in the installation compartment (9), in the ventilation system (7) and/or outside the cooking device (100), and/or
at least one gas sensor array (2) is integrated into the inner wall of the cooking chamber (1), of the installation compartment (9) and/or in the outer wall of the cooking device (100), wherein
preferably at least one gas sensor array (2) is integrated into at least two inner walls of the cooking chamber (1).

11. A cooking device (100) according to Claim 9 or 10, **characterized in that**
the memory unit, the evaluation unit and/or the control unit is/are arranged in the installation compartment (9), preferably integrated into a control or regulation device (102).

12. A cooking device (100) according to any one of Claims 9 to 11, **characterized in that**
the temperature, the temperature gradient or the temperature profile can be varied before or during a cooking process or cleaning process.

13. A cooking device (100) according to any one of Claims 9 to 12, **characterized in that**
at least one thermocouple and/or at least one heating element is/are assigned to a sensor, sensor segment and/or section of the gas sensor array (2) and/or can be controlled by means of the control unit, and/or each valve (4) can be controlled by means of the control unit.

14. A cooking device (100) according to any one of Claims 9 to 13, **characterized in that** said appliance is a hot-air steamer.

15. A method for cooking products to be cooked using a cooking appliance according to any one of Claims 9 to 14, **characterized in that**
at least the cooking chamber atmosphere is supplied to at least one sensor, sensor segment or section of at least one gas sensor array and is detected at intervals or continuously as cooking proceeds, the evaluation unit compares the result of the analysis with a standard stored in the memory unit, and the cooking process is controlled in accordance with the result of the analysis.

16. A method according to Claim 15, **characterized in that**
the results of the analysis do not differ or only differ within a predefined range from a standard that has been selected.

17. A method according to Claim 15 or 16, **characterized in that**
the temperature of the sensor, sensor segment or section and/or the standard used for comparison is/are varied, in particular before or during the cooking process.

18. A method according to any one of Claims 15 to 17, **characterized in that**
in a training phase, standards are stored in the form of profiles or patterns of the signals detected by each gas sensor array, in particular in accordance with the type of products to be cooked, the amount of products to be cooked, the quality of the products to be cooked and/or the desired degree of cooking, preferably for different temperatures of each sensor, sensor segment and/or section.

19. A method according to any one of Claims 15 to 18, **characterized in that**
once products to be cooked are placed inside the cooking appliance, in particular during a first warming-up phase, the type and/or the initial state of the products to be cooked is/are determined by means of the gas sensor array(s).

20. A method according to Claim 19, **characterized in that**
the type that has been determined and/or the initial state that has been determined is/are taken into account for control of the cooking process.

21. A method according to Claim 19 or 20, **characterized in that**
the cooking process is stopped and/or a warning signal is output if the initial state of a product to be cooked is assessed as being no longer fit for consumption.

22. A method according to any one of Claims 15 to 21, **characterized in that**
a cooking program is assigned to each standard in a training phase.

## Revendications

1. Système de prélèvement d'échantillons d'un appareil de cuisson, comprenant au moins un réseau de capteurs de gaz, auquel une atmosphère peut être amenée par l'intermédiaire d'au moins une conduite en vue de l'enregistrement d'échantillons de signal,
au moins un premier réseau de capteurs de gaz (2) destiné à une détection de l'atmosphère provenant d'un espace de cuisson (1) de l'appareil de cuisson (100),
un deuxième réseau de capteurs de gaz (2) destiné à une détection de l'atmosphère issue d'un espace d'installation (9) de l'appareil de cuisson (100),
un troisième réseau de capteurs de gaz (2) destiné à une détection de l'atmosphère issue d'un système de ventilation (7) de l'appareil de cuisson (100) et/ou un quatrième réseau de capteurs de gaz (2) destiné à une détection de l'atmosphère (11) entourant l'appareil de cuisson (100), **caractérisé par**
au moins une première conduite (20) pour l'atmosphère issue de l'espace de cuisson (1) vers les premier, deuxième, troisième et/ou quatrième réseaux de capteurs de gaz (2), au moins une deuxième conduite (24) pour l'atmosphère issue de l'espace d'installation (9) vers les premier, deuxième, troisième et/ou quatrième réseaux de capteurs de gaz (2),
au moins une troisième conduite (22) pour l'atmosphère issue du système de ventilation (7) vers les premier, deuxième, troisième et/ou quatrième réseaux de capteurs de gaz (2) et/ou au moins une quatrième conduite (26) pour l'atmosphère (11) entourant l'espace de cuisson (100) vers les premier, deuxième, troisième et/ou quatrième réseaux de capteurs de gaz (2),
où
au moins un clapet (4) commandable est agencé au niveau de l'entrée des première, deuxième, troisième et/ou quatrième conduites (20, 22, 24, 26).

2. Système de prélèvement d'échantillons selon la revendication 1, **caractérisé par** au moins une première dérivation (12) des premier, deuxième, troisième et/ou quatrième réseaux de capteurs de gaz (2), où de préférence au moins un clapet est agencé au niveau de l'entrée de la dérivation.

3. Système de prélèvement d'échantillons selon la revendication 1 ou 2, **caractérisé par**
au moins un filtre (5, 6) devant au moins un réseau de capteurs de gaz (2), en particulier devant sa surface de mesure, et/ou dans ou au niveau de l'entrée des première, deuxième, troisième et/ou quatrième conduites (20, 22, 24, 26).

4. Système de prélèvement d'échantillons selon l'une quelconque des revendications 1 à 3, **caractérisé par**
au moins une unité de refoulement (3) en liaison avec les première, deuxième, troisième et/ou quatrième conduites (20, 22, 24, 26) pour le transport d'une atmosphère à analyser vers le/les réseau(x) de capteurs de gaz (2).

5. Système de prélèvement d'échantillons selon l'une quelconque des revendications 1 à 4, **caractérisé en ce que**
le réseau de capteurs de gaz (2) comprend plusieurs champs constitués d'un film d'oxyde métallique semi-conducteur, qui sont reliés respectivement à deux électrodes, où les champs forment une surface essentiellement continue, les électrodes présentent une configuration en forme de bande et divisent la surface continue en lesdits champs de façon que chaque champ des surfaces continues est délimité respectivement par les deux électrodes.

6. Système de prélèvement d'échantillons selon l'une quelconque des revendications 1 à 5, **caractérisé en ce que**
différents capteurs, segments de capteur et/ou champs de chaque réseau de capteurs de gaz (2) présentent différentes modifications de conductivité en fonction d'une température, d'une composition, d'un dopage et/ou d'un revêtement lors d'un contact avec des gaz réducteurs ou oxydants.

7. Système de prélèvement d'échantillons selon la revendication 6, **caractérisé en ce que**
la température de chaque capteur, segment de capteur et/ou champ du réseau de capteurs de gaz (2) peut être ajustée, de préférence un gradient de température déterminé ou un profil de température déterminé peut être conféré au réseau de capteurs de gaz (2).

8. Système de prélèvement d'échantillons selon la revendication 6 ou 7, **caractérisé en ce que**
chaque capteur, chaque segment de capteur et/ou chaque champ est en liaison fonctionnelle avec un thermo-élément et/ou un élément chauffant, de préférence commandable.

9. Appareil de cuisson (100), comprenant au moins un espace de cuisson (1), au moins un espace d'installation (9), un système de ventilation, un système de prélèvement d'échantillons selon l'une quelconque des revendications précédentes, au moins une unité de mémorisation pour mémoriser les signaux détectés par l'intermédiaire des premier, deuxième, troisième et/ou quatrième réseaux de capteurs de gaz (2), au moins une unité d'interprétation destinée au traitement des signaux détectés, et au moins une unité de commande destinée à commander des processus de cuisson ou des processus de nettoyage en fonction des signaux interprétés.

10. Appareil de cuisson selon la revendication 9, **caractérisé en ce que**
au moins un réseau de capteurs de gaz (2) est installé dans l'espace de cuisson (1), dans l'espace d'installation (9), dans le système de ventilation (7) et/ou à l'extérieur de l'appareil de cuisson (100), et/ou
au moins un réseau de capteurs de gaz (2) est intégré dans la paroi intérieure de l'espace de cuisson (1), de l'espace d'installation (9) et/ou dans la paroi extérieure de l'appareil de cuisson (100), où
de préférence au moins un réseau de capteurs de gaz (2) est intégré dans au moins deux parois intérieures de l'espace de cuisson (1).

11. Appareil de cuisson (100) selon la revendication 9 ou 10, **caractérisé en ce que**
l'unité de mémorisation, l'unité d'interprétation et/ou l'unité de commande est ou sont agencée(s) dans l'espace d'installation (9), de préférence intégrée(s) dans un dispositif de commande ou régulation (100).

12. Appareil de cuisson (100) selon l'une quelconque des revendications 9 à 11, **caractérisé en ce que**
la température, le gradient de température ou le profil de température peut être modifié avant ou pendant un processus de cuisson ou un processus de nettoyage.

13. Appareil de cuisson (100) selon l'une quelconque des revendications 9 à 12, **caractérisé en ce que**
au moins un thermo-élément et/ou au moins un élément chauffant est ou sont associé(s) à un capteur, un segment de capteur et/ou un champ du réseau de capteurs de gaz (2) et/ou peut ou peuvent être commandé(s) par l'intermédiaire de l'unité de commande et/ou chaque clapet (4) peut être commandé par l'intermédiaire de l'unité de commande.

14. Appareil de cuisson (100) selon l'une quelconque des revendications 9 à 13, **caractérisé en ce que** celui-ci représente un appareil de cuisson à vapeur et air chaud.

15. Procédé de cuisson d'un produit à cuire avec un appareil de cuisson selon l'une quelconque des revendications 9 à 14, **caractérisé en ce que**
au moins une atmosphère d'espace de cuisson est amenée à au moins un capteur, segment de capteur ou champ d'au moins un réseau de capteurs de gaz et est détectée pendant la cuisson par intervalle ou de manière continue, le résultat d'analyse est comparé dans l'unité d'interprétation avec une norme déposée dans l'unité de mémorisation, et le processus de cuisson est mis en oeuvre en fonction du résultat d'analyse.

16. Procédé selon la revendication 15, **caractérisé en ce que**
les résultats d'analyse ne s'écartent pas d'une norme sélectionnée ou seulement à l'intérieur d'une plage spécifiée.

17. Procédé selon la revendication 15 ou 16, **caractérisé en ce que**
la température du capteur, du segment de capteur ou du champ et/ou de la norme utilisée pour la comparaison est ou sont modifiés, en particulier avant ou pendant le processus de cuisson.

18. Procédé selon l'une quelconque des revendications 15 à 17, **caractérisé en ce que**
des normes sont stockées, dans une phase d'apprentissage, sous la forme de profils ou d'échantillons des signaux détectés de chaque réseau de capteurs de gaz, en particulier en fonction du type de produit à cuire, de la quantité de produit à cuire, de la qualité du produit à cuire et/ou du degré de cuisson souhaité, de préférence pour différentes températures de chaque capteur, segment de capteur et/ou champ.

19. Procédé selon l'une quelconque des revendications 15 à 18, **caractérisé en ce que**
après introduction du produit à cuire dans l'espace intérieur de l'appareil de cuisson, en particulier pendant une première phase de chauffage, le type et/ou l'état initial du produit à cuire est ou sont déterminé(s) par l'intermédiaire du ou des réseau(x) de capteurs de gaz.

20. Procédé selon la revendication 19, **caractérisé en ce que**
le type déterminé et/ou l'état initial déterminé est ou sont pris en compte par la commande du processus de cuisson.

21. Procédé selon la revendication 19 ou 20, **caractérisé en ce que**
lorsque l'état initial d'un produit à cuire est qualifié de corrompu, le processus de cuisson est stoppé et/ou un signal d'avertissement est émis.

22. Procédé selon l'une quelconque des revendications 15 à 21, **caractérisé en ce que**
dans une phase d'apprentissage, un programme de cuisson est associé à chaque norme.
